# EUROPEAN PATENT SPECIFICATION

(11) **EP 1 428 878 B1**
(45) Date of publication and mention of the grant of the patent: **20.08.2008**
(21) Application number: 02027952.7
(22) Date of filing: 13.12.2002
(51) Int. Cl.: C12N 15/11, C07K 14/505

(54) **Process for the production and purification of erythropoietin**
Verfahren zur Herstellung und Reinigung von Erythropoietin
Procédé pour la préparation et la purification de l'érythropoiétine

(43) Date of publication of application: 16.06.2004
(73) Proprietor: Bioceuticals Arzneimittel AG, 61118 Bad Vilbel (DE)
(72) Inventor: Bavand, Michael, CH-5000 Arau (CH); Blasey, Horst, CH-4665 Oftringen (CH)
(74) Representative: Maiwald Patentanwalts GmbH

(56) References cited:
- WO-A1-86/07594
- WO-A1-95/07703
- WO-A1-96/32413
- WO-A1-96/35718
- WO-A1-03/045996
- E WATSON & F YAO: "Capillary electrophoretic separation of human recombinant erythropoietin (r-HuEPO) glycoforms" ANALYTICAL BIOCHEMISTRY, vol. 210, 1993, pages 389-393, XP002231297 ACADEMIC PRESS INC. NEW YORK., US ISSN: 0003-2697
- S KISHINO & K MIYAZAKI: "Separation methods for glycoprotein analysis and preparation" JOURNAL OF CHROMATOGRAPHY B : BIOMEDICAL APPLICATIONS., vol. 699, no. 1-2, 1997, pages 371-381, XP004095004 ELSEVIER SCIENCE PUBLISHERS, NL ISSN: 0378-4347
- DATABASE CA [Online] CHEMICAL ABSTRACTS SERVICE, COLUMBUS, OHIO, US; BELYAKOV, N. V. ET AL: "Method of purification of human recombinant erythropoietin" retrieved from STN Database accession no. 135:247184 CA XP002245310 & RU 2 145 610 C1 (OBSHCHESTVO S OGRANICHENNOI OTVETSTVENNOST'YU "PROTEINOVYI KONTUR", RU) 20 February 2000
- DATABASE CA [Online] CHEMICAL ABSTRACTS SERVICE, COLUMBUS, OHIO, US; MORO, ANA MARIA ET AL: "Purification Process for Erythropoietin" retrieved from STN Database accession no. 135:207867 CA XP002245311 & BR 9 900 437 A (FUNDACAO BUTANTAN, BRAZIL) 12 September 2000 (2000-09-12)
- DATABASE CA [Online] CHEMICAL ABSTRACTS SERVICE, COLUMBUS, OHIO, US; INOUE, NOBORU ET AL: "An improved method for the purification of human erythropoietin with high in vivo activity from the urine of anemic patients" retrieved from STN Database accession no. 121:74034 CA XP002245312 & BIOLOGICAL & PHARMACEUTICAL BULLETIN ( 1994 ), 17(2), 180-4 , 1994,
- DATABASE CA [Online] CHEMICAL ABSTRACTS SERVICE, COLUMBUS, OHIO, US; GHANEM, A. BEN ET AL: "Purification and biological activity of a recombinant human erythropoieti produced by lymphoblastoid cells" retrieved from STN Database accession no. 121:1934 CA XP002245313 & PREPARATIVE BIOCHEMISTRY (1994), 24(2), 127-42 , 1994,

## Description

The present invention relates to the production of recombinant glycoproteins, in particular recombinant human erythropoietin, in mammalian cells, as well as methods of purifying the produced glycoproteins from the cell culture.

With few exceptions, the secreted and membrane-associated proteins of all eucaryotic cells are glycosylated. It is well known that the carbohydrate moieties of such glycoproteins are important for exerting their biological activities and functions, such as secretion, biological half-life, antigenicity and stability. The majority of the current and potential recombinant therapeutics for human applications are glycoproteins. Modification of the glycomoiety of a recombinant human protein may lead to a product with improved pharmacokinetic and pharmacodynamic features, such as increased half-life in the circulation, targeting to the site of action (increased bioactivity and specificity) as well as increased stability *in vivo* and *in* vitro, decreased heterogeneity and decreased antigenicity/immunogenicity. Structure-function relationships for the glycomoieties have been shown for a number of therapeutic recombinant human glycoproteins, such as erythropoietin, Factor VIII and β-glucocerebrosidase.

In general, no specific biological function is found to be associated with a single, defined carbohydrate structure. Rather, structure-function relationships must be searched for in the context of analytical glycosylation patterns and relative abundance of carbohydrate chains. These in turn are characterized by physico-chemical properties such as number and type of branching structure (antennarity), repeat structures and sialic acid content. As a result of extensive structure-function evaluations of a few model proteins, few rules have emerged to classify the quality of a recombinant glycoprotein based on features of its glycomoiety.

Erythropoietin is a well-studied glycoprotein hormone involved in the maturation of erythroid progenitor cells into erythrocytes. It is essential in regulating levels of red blood cells in circulation. Naturally occurring erythropoietin is produced by the liver during fetal life and by the kidney of adults and circulates in the blood and stimulates the production of red blood cells in bone marrow. Anemia is almost invariably a consequence of a renal failure due to decreased production of erythropoietin from the kidney.

Recombinant erythropoietin produced by genetic engineering techniques involving the transfer and expression of a gene encoding erythropoietin in a mammalian host cell has been found to be effective when used in the treatment of anemia resulting from chronic renal failure. Erythropoietin is a glycoprotein with a molecular weight ranging from 34 to 38 kDa of which approximately 40 to 50% account for carbohydrate moieties. The bulk of the carbohydrate chains are N-linked to 3 asparagines and minor carbohydrates are O-linked to a serine. Complex type glycosylation with high antennary glycostructures can be found with erythropoietin and the terminal galactose is highly sialylated. The glycosylation or sialylation does not seem to be a prerequisite for receptor binding and activity of erythropoietin *in vitro,* however deglycosylated or desialylated erythropoietin exerts a short half-life *in vivo.* Thus, features of erythropoietin glycosylation and sialylation are required for enhancement of *in vivo* half-life and also influence the route of clearance and the organ targeting of the molecule.

The provision of suitable glycostructures attached to the erythropoietin protein must be the focus of development efforts for obtaining a therapeutically feasible erythropoietin preparation. Besides half-life, clearance route and organ targeting, physico-chemical studies have shown that glycosylation also increases stability of erythropoietin against chemical and biological degradation. The glycosylation and sialylation of both natural erythropoietin (urinary erythropoietin) and recombinant erythropoietin has been extensively studied at the descriptive analytical level, however only little data are available to correlate analytical data with therapeutic outcome. From available data it seems that the following features seem to be desirable in order to obtain a good product that is therapeutically effective.

First, the quality of the erythropoietin product seems to be influenced by the kind and degree of sialylation of terminal galactose. Imai et al. (1990, Eur. J. Biochem. 194:457-462) showed that erythropoietin isoforms with high sialic acid content exerted higher specific activity than those with lower sialic acid content. Rush et al. (1995, Anal. Chem. 67:1442-1452) described O-acetylation of sialic acid residues of erythropoietin and their effect on increasing circulation time. Clearance studies performed by Fukuda et al. (1989, Blood 73:84-89) in rats showed that erythropoietin derived from Chinese Hamster Ovary (CHO) cells was cleared from the blood with a half-life of approximately 2 hours, following a bi-phasic clearance mechanism. Asialo erythropoietin was cleared within ten minutes, suggesting a galactose-binding mechanism of hepatic cells being responsible for clearance of asialo erythropoietin. Spivak and Hogans (1989, Blood 73:90-99) showed that with commercial erythropoietin, plasma clearance of erythropoietin was multiexponential with an initial rapid distribution phase (T_{1/2}=53 min.) and a slower elimination phase (T_{1/2}=180 min.). Organ accumulation in kidneys, bone marrow and spleen started 30 min. after injection of commercial erythropoietin. Asialo erythropoietin was cleared rapidly via the hepatic route.

Therefore, published evidence on the sialylation of erythropoietin revealed three main effects on sialylation of erythropoietin on *in vivo* activity:
- Clearance of erythropoietin in the circulation decreases when erythropoietin is sialylated. The extent of sialylation depends on the extent of antennary structures present that provide the substrate for sialylation of terminal galactoses.
- Erythropoietin isoforms with the highest number of sialic acids seem to exert the highest specific activity.
- Asialo erythropoietin is rapidly cleared via the hepatic route, low sialylated erythropoietin (biantennary structures ) are targeted to kidneys and spleen. Sialylated higher antennary structures (triantennary and tetraantennary) are mainly targeted to bone marrow cells.
- A high degree of O-acetylation of sialic acid residues increases circulation time by reducing hepatic clearance.

It is therefore desirable to use conditions during mammalian cell culture that consistently allow maximal sialylation of the majority of N-linked carbohydrate structures. Conditions for maximal sialylation would be a considerable advantage for the manufacture of glycoprotein therapeutics. Given the beneficial effects of highly sialylated glycoproteins, in particular erythropoietin, it is an object of the present invention to provide methods of increasing the sialylation level of glycoproteins, in particular of erythropoietin, by modification of cell culture conditions during production of glycoproteins.

Surprisingly, it has been found that the degree of sialylation of glycoproteins, in particular of erythropoietin, can be optimised by culturing the glycoprotein-producing mammalian cells at pH that supports the production of highly sialylated proteins.

Traditionally, the pH of a culture medium is manipulated with the goal of achieving maximum cell growth and/or production. Media for cell culture are typically prepared with pH adjusted to 7,4, similar to the pH of human blood. The optimum pH is however cell line dependent and can vary from pH 6,9-7,6 (reviewed in Eagle (1973) J. Cell Physiol. 82:1-89).

Thus, the effect of pH on cell growth and/or production, and not its effect on the nature of a cell product has been the determining factor in selecting pH values in traditional cell culture. For example, the prior art has disclosed varying the pH, glucose, and lactate production in the growth and production phases of the cell culturing process in order to optimise growth and production, respectively (see for example WO 88/01643, which reference does not address the nature of the cell products produced). US patent 5,096,816 discloses the effect of ammonia on the glycosylation of cell products through pH control. The preferred method describes a multi-pH strategy for optimum cell growth at higher pH and then the use of low pH to minimize the inhibition of terminal sialylation of cell products by high levels of ammonia.

Surprisingly, we have now found an opposite effect than described in US patent 5,096,816. Production of the desired cell product containing a maximum degree of sialylation is best in the higher pH range.

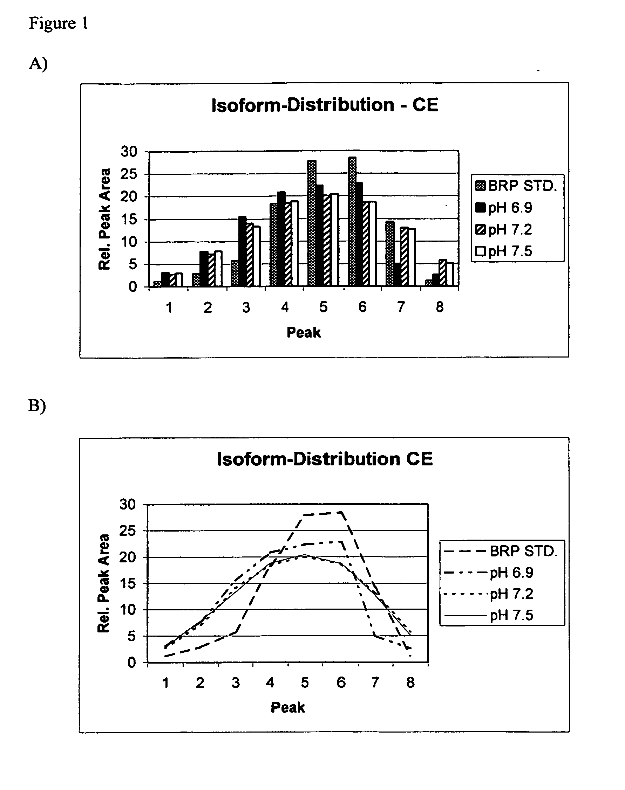

As shown in Figure 1, cell culture employing pH control between pH 7.2 - 7.5 results in product formation with a higher degree of sialylation. Figures 1A and 1B show results from product isoform distribution profiles of recombinant human erythropoietin obtained after cultivation under different pH-controlled conditions. The product produced under the different pH culture conditions was isolated by a 2-step purification procedure (product capture with a Q-Sepharose XL (Amersham Lifesciences, Germany) and salt step elution followed by a Hydrophobic Interaction Chromatography (HIC) step with Butyl Sepharose FF(Amersham Lifesciences, Germany) and analysed by capillary electrophoresis (CE). CE separates erythropoietin isoforms by charge and is therefore a suitable analytical tool for assessing the sialylation status of a cell product.

As can be seen in Figure 1A, there is a shift of the isoform distribution towards the higher charged isoforms (higher peak numbers) at culture conditions employing a pH of 7.2-7.5. A purified preparation of a highly glycosylated and sialylated reference preparation, the European Pharmacopoeia (Ph. Eur. (2002) 4^{th} edition) erythropoietin BRP Standard was included in the analysis, for comparison. In particular, the highest forms of sialylated erythropoietin product, represented by the isoforms in peak 7 and 8 are present in a significantly lower proportion in products derived from the cell culture at pH 6.9. Thus, cell culture employing a pH control of between pH 7.2-7.5 results in product formation with a higher degree of sialylation of products. Preferred is the pH 7.2, which yielded a good product yield and a high degree of sialylation. Even though the overall cell density at this pH is lower than e.g. for pH 6.9, the quality of the glycoprotein (in this case erythropoietin) with respect to the sialyl-content is better at a pH between 7.2 and 7.5.

Moreover, the surprising finding that cell cultivation at comparably high pH values such as pH 7.2 to 7.5 leads to erythropoietin with a higher content of glycosyl- and sialyl-groups even though cell mass is reduced can be extended to the production of other glycoproteins such as t-PA, Factor XIII, Factor VIII, Interferon-β, IL-2.

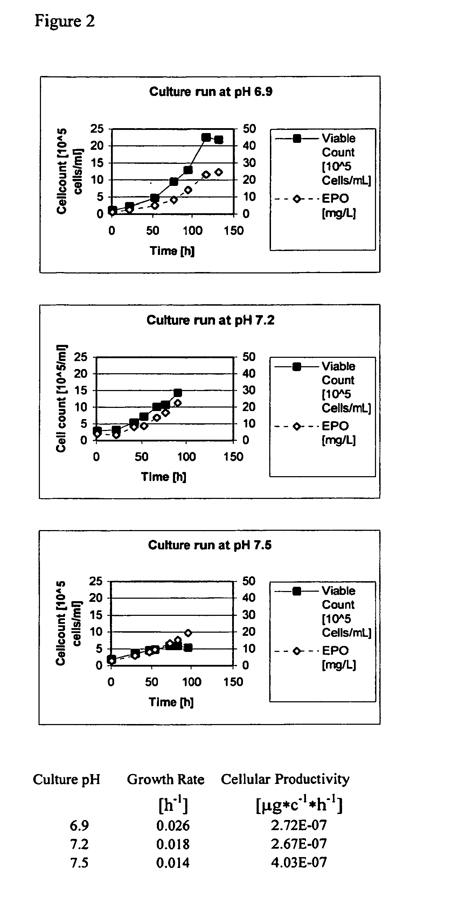

The optimal pH value may be cell type and cell line specific and can be determined by experiments as set out in Figure 2. In the case of CHO cells it will be pH 7.2 as exemplified by the EPO producing CHO cells.

The term "isoform", as used herein, refers to a erythropoietin preparation/fraction that contains glycoproteins which have identical or very similar amino acid sequence and a common isoelectric point but which may differ in respect to the extent, to the complexity, to the nature, to the antennarity and to the order of attached gylcosyl- and sialyl-groups. Isoforms according to the invention may also comprise multiple erythropoietin forms of the same or very similar amino acid sequence and isoelectric point which differ additionally in other charge carrying modifications such as acetylation and sulfatation. The term "very similar amino acid sequence" indicates that the amino acid sequence of a protein also comprises those sequences that are functionally equivalent to the wild type amino acid sequence.

Thus, glycoprotein isoforms according to the invention are defined by their isoelectric point and amino acid sequence and each isoform may therefore actually comprise multiple isoforms in the strict chemical sense.

The present invention also relates to a method for the purification of highly glycosylated and particularly sialylated glycoproteins, in particular recombinant human erythropoietin, from cell culture.

Recombinant human erythropoietin, when analysed from cell culture supernatant by isoelectric focussing (IEF), shows a broad isoelectric point (pI) isoform profile with more than 14 different isoforms over a range of pH 3-9 (Gokana et al. (1997) J. Chromatogr., 791 (1-2): 109-18). The erythropoietin isoforms arise mainly due to their variant glycosyl content with variant numbers of negatively charged terminal sialic acid residues. Erythropoietin forms with more sialic acid residues and thus a more acidic pI profile are thought to be of higher therapeutic value because terminal sialic acid residues on glycostructures prevent the fast clearance *in vivo* via the asialo-receptor route.

International patent application WO-A 1-86/07594 discloses a method of purifying erythropoietin from a fluid comprising the steps of subjecting said fluid to reverse phase liquid chromatographic separation involving an immobilized C₄ or C₆ resin, thereby to selectively bind erythropoietin in said fluid to said resin, followed by a selective elution of bound erythropoietin from said resin with an aqueous ethanol solution of from 50 to 80 percent at a pH of from about 4.5 to 8.0, and isolating erythropoietin-containing fractions of the eluent.

International patent application WO-A1-96/35718 describes a preparation of protein having erythropoietin activity which is obtained from serum-free cell culture medium using a purification process involving dye chromatography, hydrophobic chromatography on an alkylated or arylated carrier, chromatography on hydroxyapatite, hydrophobic chromatography and anion exchange chromatography.

Inoue N. et al. (1994, Biol. & Pharm. Bulletin 17(2):180-184) describe a method for the purification of human erythropoietin with high *in vivo* activity form urine which involves ionexchange, gel permeation, affinity chromatography, and reverse phase chromatography but no stabilizing procedures.

It is therefore an object of the invention to develop a downstream purification process (DSP) wherein in general acidic isoforms of glycoproteins, in particular acidic isoforms of erythropoietin, are enriched.

According to the invention "acidic isoforms" of erythropoietin comprise those isoforms that have a high degree or content of glycosyl-, and preferably of sialyl-groups.

A higher degree of sialylation of a glycoprotein can be generally achieved if an increasing number of sugar chains is terminated by sialyl-groups. Thus, if the overall glycosyl-content of a glycoprotein is increased, a higher degree of sialylation can be achieved as there may be more sugar chains available for sialylation.

According to the invention, erythropoietin has a high degree or content of glycosyl- and preferably-sialyl groups if they show an isoform pattern comparable to the respective standards of the Eur. Ph. such as the Eur. Ph. BRP erythropoietin standard as determined by IEF.

According to the invention the term "complex protein mixture" refers to a mixture comprising a multitude of different proteins and isoforms thereof such as e.g. cellular extracts.

Protein products are generally isolated from biological culture fluids employing a sequence of purification steps. Each purification step contains one or several wash steps. Washing conditions generally do not provide for high selectivity for quantitative separation of related impurities (e.g. protein isoform impurities) from the desired product molecules. Purification methods with higher selectivity, such as chromatofocussing employ pH-gradients using ampholytes, however they are generally not suitable for large-scale manufacturing due to their high costs and limited scaleability. Single salt wash steps provide a limited selectivity for the enrichment of the desired molecules. Harsh wash steps will provide an enrichment for the desired molecules, however at the cost of an overall loss of yield.

It has now surprisingly been found that erythropoietin isoforms with a low or acidic isoelectric point, i. e. with high content of sialyl-moieties, can be selectively enriched by a DSP process if anion exchange chromatography is combined with at least one acidic wash step during the purification procedure.

Thus, according to the present invention, one way to overcome the limited selectivity of salt wash steps is to use at least one acidic wash step, thereby providing for a higher overall selectivity of the purification procedure.

The purification process according to the invention employs multiple anion chromatography steps with at least one acidic wash step in the DSP process, yielding a high selectivity towards the selection of acidic protein isoforms from a pool of protein isoforms.

In another preferred embodiment of the invention the anion chromatography capture step is followed by an acidic wash step and other chromatography steps of different principle such as HIC or affinity chromatography. This may allow decreasing impurities and proteins that co-purify with the anion chromatography capture step. The person skilled in the art understands that the sequence and the nature of the chromatography steps after the anion chromatography capture step depend on the glycoprotein to be purified and can be easily determined by the person skilled in the art.

In an even more preferred embodiment of the invention, an anion chromatography capture step and an optional acidic wash step are followed by chromatography steps of different principle. In a last step, another anion exchange chromatography and a mandatory acidic wash step are then applied to selectively enrich the preparation for glycoproteins with high glycosyl- and sialyl-content.

Accordingly, the DSP process of the invention for the purification of recombinant human erythropoietin isoforms with a high glycosyl- and sialyl-content, from cell culture fluids, comprises the following purification procedure:
a) an anion exchange chromatography step as a capture step and an acidic wash step.

Preferably the process for the purification of recombinant human erythropoietin isoforms with a high sialyl-content, from cell culture fluids comprises the following steps:
a) a first anion exchange chromatography step as a capture step and an acidic wash step,
b) chromatography steps based on a principle other than anion exchange chromatography such as HIC and affinity chromatography.

Another preferred method for the purification of recombinant human erythropoietin isoforms with a high glycosyl- and sialyl-content, from cell culture fluids, comprises the following steps:
a) a first anion exchange chromatography step as a capture step,
b) chromatography steps based on a principle other than anion exchange chromatography, such as HIC and affinity chromatography purification,
c) a second anion exchange chromatography step,
wherein at least one acidic wash step is combined with substep a) and/or substep c).

An even more preferred process for the purification of recombinant human erythropoietin isoforms with a high glycosyl- and sialyl-content, from cell culture fluids, comprises the following steps:
a) a first anion exchange chromatography step as a capture step and an optional acidic wash step,
b) HIC,
c) affinity chromatography,
d) a second anion exchange chromatography step and an acidic wash step.

The hydrophobic interaction chromatography of step b) is preferably using a butyl sepharose chromatography resin. The affinity chromatography of step c) is preferably using a hydroxyapatite chromatography resin. The order of substeps b) and c) may also be reversed. Elution in substeps a) to d) may be performed as step or gradient elution, depending on the specific circumstances.

In the following, the different steps of the DSP process will be described in detail.

### Sample preparation:

Cell culture fluids were generally from fermentations, performed at the preferred pH 7.2, yielding highly sialylated glycoprotein, thus qualifying for further acidic pI isoform enrichment by the above-specified sequence. A preferred embodiment for cell culture fluid preparation is given in the example section.

Generally cell culture fluids or generally complex protein mixtures were concentrated by ultra-filtration and dialysed against a buffer that allows for binding of the glycoprotein to the anion exchange resin.

### Step a) Anion exchange chromatography as capture step

### Resins

Anion exchange chromatography as capture step can be performed with all common commercially available anion exchange resins or membranes. Anion exchange resins may be used in the form of pre-poured columns. Alternatively columns may be self-prepared.

There are no specific limitations as to the capacity and the geometry of the columns other than the usual ones. The person skilled in the art knows that the amount of anion exchange resin to be used depends on the overall protein content of the cell culture fluid applied to the column in the capture step.

Typical anion exchange resins that can be used for the purpose of the invention comprise functional groups such as:
- Diethylaminoethyl (DEAE) moieties, resins include e.g. DEAE-Sepahrose (Amersham Biosciences, Germany), Macroprep DEAE support (Bio-Rad, Germany), Fractorgel EMD DEAE (Merck, Germany) and others
- Quaternary aminoethyl (QAE) moieties, resins include e.g. Toyopearl QAE (Toyo Biosep, *Germany*) and others.
- Quaternary ammonium (Q) moieties, resins include e.g. Q-Sepahrose XL (Amersham Biosciences, Germany), Q-Sepharose FF (Amersham Biosciences, Germany), Resource Q (Amersham Biosciences, Germany), Macro Prep High Q (Biorad, Germany), Toyopearl Super Q (Toyo Biosep, Germany)
- Dimethylaminoethyl (DMAE) moieties, resins include e.g. Fractogel EMD DMAE (Merck AG, Germany)
- Trimethylaminoethyl (TMAE) moieties, resins include e.g. Fractogel EMD TMAE (Merck AG, Germany)
- Sartobind Membraneadsorber (MA) Q100 (Sartorius, Germany)

### Temperature

The capture can be usually performed between 2°C and ambient temperature. Preferably it is performed at 2 - 8°C.

### Buffer composition

Equilibration, washing and elution buffers may comprise all buffer types usually used for anion exchange chromatography (Deutscher (1990) Protein Purification, Academic Press, NY). Thus, buffering agents comprise Na-Phosphate, Na-Acetate Tris/HCl, HEPES, or other suitable buffering agents. Moreover buffers may contain between 5 mM and 1 M NaCl, KCl, or other suitable salts, depending on whether the buffer is used for equilibration, washing or elution. Equilibration and washing buffers contain lower concentrations of the aforementioned salts than elution buffers. Buffers may also contain between 1 and 10 mM MgCl₂ or CaCl₂. Additionally buffers may contain between 0.5 and 10 mM EDTA and other protease inhibitors such as Phenylmethanesulfonyl fluoride (PMSF). The pH of the buffers will be usually adjusted to a pH between 6.8 and 7.8, preferably between 7.0 and 7.6 and more preferably between 7.2 and 7.5 and even more preferably to pH 7.5. For buffer composition of buffers used for acidic wash steps see below.

Preferably equilibration and washing buffers contain between 5 - 50 mM Na-Phosphate pH 7.5 and more preferably 20 mM Na-Phosphate pH 7.5. Elution buffers contain between 150 - 500 mM NaCl and 5 - 50 mM Na-Phosphate pH 7.5 and preferably 300 mM NaCl, 20 mM Na-Phosphate pH 7.5.

### Processing:

Equilibration of columns is usually carried out with 5 to 20 column volumes (CV) of equilibration buffer. Washing of columns after loading is usually carried out with 2 to 5 CV washing buffer. Elution is usually carried out by a step elution with 2 to 5 CV elution buffer.

The flow rate of the various steps depends on the geometry of the columns and the resin used. It can be calculated according the manufacture's instructions or general principles. The flow rate during elution is generally lower than the flow rate during equilibration, loading and washing.

### Step b) Hydrophobic Interaction Chromatography (HIC)

Material eluted from the capture step contains usually high concentrations of salt (between 100 and 500 mM NaCl or KCl). Therefore a useful continuation as the next step is hydrophobic interaction chromatography (HIC) as the first polishing step. The eluate from the capture step is loaded after it has been diluted, usually with phosphate buffer and isopropanol.

HIC can be performed with all common commercially available HIC resins. HIC may be used in the form of pre-poured columns. Alternatively columns may be self-prepared.

There are no specific limitations as to the capacity and the geometry of the columns other than the usual ones. The person skilled in the art knows that the amount of HIC resin to be used depends on the overall protein content of the protein preparation applied to the column.

### Resins

HIC-resins that can be used for the purpose of the invention comprise matrices such as Butyl-, Phenyl-, Propyl or Octyl- Sepharose (Amersham Bioscience, Germany), Macro-Prep Methyl or t-butyl HIC support (Biorad, Germany) or Fractogel EMD with Propyl or Phenyl ligands (Merck AG, Germany). Preferred hydrophobic interaction chromatography material is Butyl-sepharose.

### Temperature

HIC can be usually performed between 2 and 35°C. Preferably it is performed at 15 - 25°C. In the case of erythropoietin the temperature should not be lower than 18°C, since erythropoietin does not bind quantitatively to the HIC resin at lower temperatures.

### Buffer composition

Equilibration, washing and elution buffers may comprise all buffer types usually used for HIC (Deutscher (1990) Protein Purification, Academic Press, NY). Thus, buffering agents comprise Na-Phosphate, Na-Acetate, Tris/HCl, HEPES, or other buffering agents. Moreover, buffers may contain between 0,5 mM and 3 M NaCl, KCl or other suitable salts depending on whether the buffer is used for equilibration, washing or elution. Equilibration and washing buffers contain higher concentrations of the aforementioned salts than elution buffers.

The equilibration- and the washing buffer contain up to 30%, preferably up to 10% of an alcohol such as Isopropanol. The elution buffer contains up to 50%, preferably up to *19%* of an alcohol such as Isopropanol.

Buffers may also contain between 1 and 10 mM MgCl₂ or CaCl₂. Additionally buffers may contain between 0.5 and 10 mM EDTA and other protease inhibitors such as Phenylmethanesulfonyl fluoride (PMSF).

The pH of the dilution, equilibration and washing buffers will be usually adjusted to a pH between 6.8 and 7.8, preferably between 7.0 and 7.7, more preferably between 7.2 and 7.6 and even more preferably to pH 7.5. The pH of the elution buffer will be usually adjusted to a pH between 6.5 and 7.5, preferably between 6.7 and 7.2 and even more preferably to pH 6.9.

The dilution buffer preferably contains between 2 - 6 M NaCl, 5 - 50 mM Na-Phosphate pH 7.5 and more preferably 4 M NaCl, 20 mM Na-Phosphate pH 7.5. Preferably equilibration und washing buffers contain between 1 - 2.5 M NaCl, 5 - 50 mM Na-Phosphate pH 7.5, 5 - 15 % Isopropanol and more preferably 2 M NaCl, 20 mM Na-Phosphate pH 7.5, 10% Isopropanol. Elution buffer contains between 0.5 - 1 M NaCl, 5 - 50 mM Tris/HCl, pH 6.9, 10 - 50 % Isopropanol, 5 - 10 mM CaCl₂ and preferably 0.75 M NaCl, 20 mM Tris/HCl, pH 6.9, 19 % Isopropanol, 5 mM CaCl₂.

### Processing:

Equilibration of columns is usually carried out with 5 to 15 column volumes (CV) of equilibration buffer. Washing of columns after loading is usually carried out with 3 to 7 CV washing buffer. Elution is usually carried out by a step elution with 2 to 5 CV elution buffer.

The flow rate of the various steps depends on the geometry of the columns and the resin used. It can be calculated according the manufacture's instructions or general principles.

### Conductivity:

Adjustment of the load conductivity was identified to be critical in order not to lose erythropoietin or other glycoproteins in the flow through. According to the invention conductivity of load during HIC should be comparable to that in the equilibration buffer. The conductivity in the load was typically adjusted to 95-105 mS/cm.

### Isopropanol concentration and temperature:

Formation of precipitation was observed immediately after elution from the HIC resin in some cases, maybe due to the Isopropanol content. Even if the eluate was diluted down to 10% Isopropanol or less (see below), precipitation occurred.

Thus, in the case of erythropoietin precipitation occurred after elution from the Butyl-Sepharose column (see below). Furthermore, when the eluate was diluted to 6.3% isopropanol with buffer at room temperature and stored overnight at 4°C, precipitation increased further. It is assumed that precipitation was induced by a combined solvent concentration and temperature effect. Precipitation led to substantial losses (< 30 % loss in erythropoietin).

Therefore according to the invention, eluate fractions of the HIC step containing erythropoietin or the desired glycoprotein should be immediately diluted with cold buffer to a concentration below 10 %, preferably below 7% and even more preferably below 5% Isopropanol. In the case of erythropoietin dilution to a concentration of ≤ 6.3 % isopropanol prevents formation of precipitate.

### Step c): Affinity chromatography

Affinity purification can be performed with all common commercially available affinity resins that are appropriate for purification of glycoproteins. Affinity resins may also be specifically designed for certain glycoproteins. Affinity resins may be used in the form of pre-poured columns. Alternatively columns may be self-prepared.

There are no specific limitations as to the capacity and the geometry of the columns other than the usual ones. The person skilled in the art knows that the amount of affinity resin to be used depends on the overall protein content of the protein preparation applied to the column.

### Resins

Affinity-resins that can be used for the purpose of the invention comprise matrices such as Macro-Prep (Bio-Rad, Germany), Dye-Sepharose (Biotechnol. Appl. Biochem. 29, 151-156, 1999), Peptide-ligand-affinity resins (Kaufmann D.B., Biotech. Bioeng., Vol. 77, No. 3, Feb. 5, 278-288, 2002) antibody-affinity resins (Ben Ghanem et al., Preparative Biochemistry 24(2): 127-142, 1994), lectine-affinity resins (Amersham Biosciences, Germany).

### Temperature

Affinity chromatography can be usually performed between 2°C and ambient temperature. Preferably it is performed at ambient temperature.

### Buffer composition

Equilibration, washing and elution buffers may comprise all buffer types usually used for the respective affinity resins (Deutscher (1990) Protein Purification, Academic Press, NY). Thus, buffering agents for e. g. hydroxyapatite chromatography may comprise Na-Phosphate, Na-Acetate, Tris/HCl, HEPES and other suitable buffering agents. Moreover buffers may contain between 0 and 1 M NaCl, KCl, and other suitable salts, depending on whether the buffer is used for equilibration, washing or elution. Elution buffers will comprise also between 0 and 200 mM K₂HPO₄ to form a gradient. The equilibration buffer may also contain between 1 and 10 mM MgCl₂, CaCl₂. Additionally buffers may contain between 0.5 and 10 mM EDTA and other protease inhibitors such as Phenylmethanesulfonyl fluoride (PMSF).

The pH of buffers will be usually adjusted to a pH between 6.7 and 7.8, preferably between 6.8 and 7.5, more preferably between 6.8 and 7.2 and even more preferably to pH 6.9. If HIC has preceded affinity chromatography, equilibration and the first washing buffer may comprise up to 250 mM NaCl and up to 9% Isopropanol. The second washing buffer may contain no isopropanol, NaCl and CaCl₂.

In the case of hydroxyapatite chromatography, equilibration and elution buffers preferably contain between 5 - 50 mM Tris/HCl pH 6.9 and more preferably 10 - 20 mM Tris/HCl pH 6.9. The equilibration buffer preferably also comprises 250 mM NaCl and 9% Isopropanol in the case erythropoietin is purified and HIC has preceded affinity purification. Elution buffers contain between 0 - 150 mM K₂HPO₄ and 5 - 50 mM Tris/HCl pH 6.9 and preferably 0 to 40 mM K₂HPO₄ and 20 to 16 mM Tris/HCl pH 6.9, to form a gradient.

### Processing:

Equilibration of columns is usually carried out with 5 to 20 column volumes (CV) of equilibration buffer. Washing of columns after loading is usually carried out with 2 to 6 CV washing buffer. A second washing with 1 to 2 and preferably 1 to 1.2 column volumes of a washing buffer may follow this. Elution is usually carried out by a gradient elution with 2 to 6 CV elution buffer.

The flow rate of the various steps depends on the geometry of the columns and the resin used. It can be calculated according the manufacture's instructions. The flow rate, during elution, loading and washing is usually 80 cm/h, and during equilibration usually 113 cm/h.

### Step d) Anion exchange Chromatography

As indicated above a second anion exchange chromatography step may be performed at the end of the DSP process which defines the final product isoform composition. This step includes an acidic wash step to specifically remove the EPO isoforms of more basic pI.

### Resins

Anion exchange chromatography at this stage can be performed with all common commercially available anion exchange resins or membranes. Anion exchange resins may be used in the form of pre-poured columns. Alternatively columns may be self-prepared.

There are no specific limitations as to the capacity and the geometry of the columns other than the usual ones. The person skilled in the art knows that the amount of anion exchange resin to be used depends on the overall protein content of the protein preparation applied to the column.

Generally all resins usable for the anion exchange chromatography capture step (such as those listed in step A) may be used for this second anion exchange chromatography step. As a substantial amount of proteins will have already been removed at this stage of the DSP process, anion exchange resins that allow specifically for high resolution separation of proteins may be applied.

Therefore resins as Resource Q (Amersham Biocsiences, Germany) are preferably used at this stage of the DSP process. Accordingly flow rates will generally be lower than during the anion exchange chromatography capture step (see examples).

### Temperature

The capture can be usually performed between 2° C and ambient temperature. Preferably it is performed at ambient temperature.

### Buffer composition

Equilibration, washing and elution buffers may comprise all buffer types usually used for anion exchange chromatography (Deutscher (1990) Protein Purification, Academic Press, NY). Thus, buffering agents comprise Na-Phosphate, Na-Acetate, Tris/HCl, HEPES or any suitable buffering agent. Moreover buffers may contain between 0 and 1 M NaCl, KCl, or any suitable salt, depending on whether the buffer is used for equilibration, washing or elution. Equilibration and washing buffers contain lower concentrations of the aforementioned salts than elution buffers. Buffers may also contain between 1 and 10 mM MgCl₂ or CaCl₂. Additionally buffers may contain between 0.5 and 10 mM EDTA and other protease inhibitors such as Phenylmethanesulfonyl fluoride (PMSF). The pH of the buffers will be usually adjusted to a pH between 6.8 and 7.8, preferably between 7.0 and 7.6, more preferably between pH 7.2 and 7.5 and even more preferably to pH 7.5. For buffer composition of buffers used for acidic wash steps see below.

Preferably equilibration and washing buffers contain between 5 - 50 mM Na-Phosphate pH 7.4 and more preferably 10 mM Na-Phosphate pH 7.4. Elution buffers contain between 150-750 mM NaCl and 5 - 50 mM Na-Phosphate, pH 7.5 and preferably 500 mM NaCl, 15 mM Na-Phosphate, pH 7.4.

### Processing:

Equilibration of columns is usually carried out with 5 to 20 column volumes (CV) of equilibration buffer. Washing of columns after loading is usually carried out with 5 to 15 CV washing buffer. Elution is usually carried out either by gradient or step elution with 3 to 10 CV elution buffer.

The flow rate of the various steps depends on the geometry of the columns and the resin used. It can be calculated according the manufacture's instructions. The flow rate is generally approximately 200 cm/h during equilibration, loading, washing and elution.

### Acidic wash steps at the anion exchange chromatography

According to the invention at least one acidic wash step has to be carried out during the DSP process at one of the anion exchange chromatography steps. At what stage of the DSP process this acidic wash step is to be carried out depends on the glycoprotein purified. However, if a second anion exchange chromatography step is carried out during the DSP process as it is preferred for the purification of highly sialylated forms of erythropoietin, the acidic wash step is preferably carried out at the second anion exchange chromatography step.

The general features of the acidic wash step are set out in the following.

An acidic wash step can be employed when generally a glycoprotein (specifically erythropoietin) is bound to an anion exchange matrix, the predominant binding forces being ionic interactions between the positively charged groups of the matrix and the negatively charged residues of the glycoprotein, predominantly resulting from sialic acid residues. Other glycoproteins that can be purified by this approach comprise t-PA, Factor XIII, Factor VIII, Interferon-β, IL-2.

The ionic interactions between the glycoprotein, e.g.erythropoietin, and the matrix can be modulated by pH or ionic strength. It is assumed that by lowering the pH, the sialic acid residues are gradually neutralized by protonation, so that binding of the glycoprotein, e.g. erythropoietin, to the matrix becomes weaker, eventually resulting in the elution of erythropoietin from the column. The acid procedure can be therefore used to wash off the glycoform isoforms with lower sialylation status (basic glycoprotein isoforms e.g. of erythropoietin), thus retaining glycoform isoforms with higher sialylation status (acidic glycoprotein isoforms of e.g. erythropoietin). By employing a combination of salt strength and pH, one can modulate the system further to obtain desired isoforms or to remove unwanted isoforms.

Thus, it was an object of the invention to identify acidic wash conditions (ionic strength and pH) that allow to selectively elute basic glycoprotein (e.g. erythropoietin) isoforms and unrelated impurities, whilst retaining quantitatively preferred acidic glycoprotein (e.g. erythropoietin) isoforms. According to the invention, the DSP process includes therefore at least one acidic wash step at one of the anion exchange chromatography steps.

Thus, according to the invention, acidic wash steps may be performed at step a) or at step d) only if the DSP process comprises two anion exchange chromatography steps. In another embodiment of the invention, acidic wash steps may be performed at step a) and step d) if the DSP process comprises two anion exchange chromatography steps.

Thus, the position of the acidic wash step is not restricted to any of the 2 positions of the ionexchange step. However, for purification of specific glycoproteins such as erythropoietin, it may be preferable that only one acidic wash step is performed at step d) of the DSP process. The use of at least one acidic wash step in connection with the repeated use of the same column chromatography principle (anion exchange) is particularly suitable for increasing the selectivity of separation of closely related molecules that differ by subtle charge differences.

If an acidic wash is performed at the first anion exchange chromatography step at step a) (capture step), this may serve i) to mainly remove unrelated impurities of the cell culture fluids or supernatant and ii) to remove some basic glycoprotein (e.g. erythropoietin) isoforms.

The acidic wash step at step d) (second anion exchange chromatography) serves to selectively remove specifically basic glycoprotein (e.g. erythropoietin) isoforms.

The advantages and features of the acidic wash procedure according to the invention are the following:
- too strong acidic wash steps at either step a) or step d) will cause unacceptable product losses;
- one single acidic wash at step d) with intermediate strength yields a glycoform isoform profile with a favourable enrichement of acidic pI isoforms; and
- an additional mild acidic wash step in step a) will provide a glycoprotein isoform pool that has a decreased content in basic glycoprotein isoforms and contaminating unrelated basic proteins. This pre-purified glycoprotein pool qualifies for further purification for further selective removal of basic isoforms, employing a second acidic wash. This combination yields a strong enrichment of acidic pI isoforms.

### Acidic wash step at the anion exchange capture step

Buffers for acidic wash may contain Na-phosphate, Na-Acetate, Na-Citrate, MES or other suitable buffering agents. Buffers preferred for the acidic wash step at the capture step usually comprise between 5 and 50 mM, preferably between 10 and 40 mM and even more preferably between 20 and 35 mM Na-Acetate.

Buffers may also contain between 1 and 10 mM MgCl₂ or CaCl₂. Additionally buffers may contain between 0.5 and 5 mM EDTA and other protease inhibitors such as Phenylmethanesulfonyl fluoride (PMSF).

The pH of the buffers for the acidic wash will be usually adjusted to a pH between 2.0 and 5.5, preferably between 2.5 and 4.5 and even more preferably between 3.0 and 4.0 depending on the buffering agent used and the glycoprotein to be purified.

As the ionic strength of the buffer also has an influence on the selectivity of the acidic wash step, buffers that contain Na-Acetate are preferred.

Preferably acidic washing buffers contain between 20 and 40 mM Na-Acetate pH 3.0 - 4.0 and more preferably 33 mM Na-Acetate pH 4.0.

### Acidic wash step at the second anion exchange step

An acidic wash step at the second anion exchange chromatography step is generally carried out with buffers of the same or comparable composition as buffers used for acidic wash steps at the capture step.

Preferred buffers at this second ion exchange step contain between 5 and 30 mM Na-Acetate pH 3.0 - 4.0 and more preferably 20 mM Na-Acetate pH 4.0.

### Examples

Examples are given below that illustrate preferred embodiments of the invention for the purification and enrichment of highly glycosylated and sialylated isoforms of erythropoietin. The examples should not be interpreted as to restrict the invention.

### Expression system for production of erythropoietin

Erytropoietin was expressed with a DHFR⁻ Chinese Hamster Ovary suspension cell line (CHO, Puck et al. (1958) J. Exp. Med. 108: 945; Urlaub et al. (1980) PNAS USA 77: 4216-4220). The Dihydrofolatereductase (DHFR) negative host cell line was adapted to grow in protein-free medium. The erythropoietin gene was subcloned into a DHFR gene carrying vector under the control of a strong cytomegalovirus promotor. Transfected cells were selected and the erythropoietein gene amplified in the presence of methotrexate. Following a two-round of cell cloning, clones were selected for good growth, high product titre and production of erythropoietin with a favourable glycosylation pattern. Glycosylation pattern of erythropoietin recombinant cell lines were determined by mapping of N-linked oligosaccharides with HPAD (Hermentin et al. (1996) Glycobiology, 6 (2): 217-30) and HPLC (Kanazawa (1999) Biol. Pharm. Bull 22 (4) 339-346).

The clone, which was selected for further studies, grew (in batch) at a doubling time of approximately 22 hours and produced approximately 45 mg/l erythropoietin at a cell density of 2x10⁶ cells/ml with a high antennary glycostructure.

### Culture conditions

The recombinant CHO cells which were producing erythropoietin were grown at the 2-litre scale and at the 20-litre scale in a) batch, b) repeated-batch and c) repeated-fed-batch processes. Fermentors were inoculated at a cell density of approximately 2x10⁵ cells per ml. In batch cultures, cells were grown into the plateau phase and harvested at approximately 2x10⁶ cells per ml (product concentration at approximately 45mg/l).

In a repeated batch process, cells were grown to approximately 1.5-2x10⁶ cells per ml (product concentration approximately 20 mg/ml) and the culture was split to approximately 2x10⁵ cells per ml by removing a part of the culture and replacing the withdrawn culture volume with fresh medium to initiate the subsequent production cycle. The removed culture containing the product was purified.

In the repeated-fed-batch process, the cultivation time of the individual cycles of the repeated batch culture could be extended 1 day by feeding a concentrate of nutrients to the culture. The harvest was performed 4 days after the start at approximately 2-3x10⁶ cells per ml (product yield was approximately 30-40 mg/l). Other processes (e.g. continuous or perfusion cultivation) are feasible alternatives.

Medium for fermentation was a protein free medium, typically used for culturing CHO cells, which was supplemented with 2 mM glutamin, 0.1% Pluronic F68 (Sigma, Germany) and 2 ppm antifoam C (Sigma, Germany). Temperatures were between 34°C and 37°C, preferably 37°C. The pH was between pH 7.2 and pH 7.5, preferred was pH 7.2. During cultivation, correction of the pH in the bicarbonate buffered medium was done by CO₂ injection or NaOH addition. Oxygen concentration was controlled at 40% air saturation by sparging the culture with air, nitrogen and oxygen.

The culture harvests were cleared from most of the cells (98%) by continuous centrifugation with a Biofuge 17RS (at approximately 3540 x g). Remaining cells and debris were separated by filtration with a Sartocon II (Sartorius, Germany) and a 0.6 m² Cellulose Acetate Filtration-Module 0.2 µm (Nr. 3021230706W, Sartorius, Germany).

The filtrate was concentrated by ultrafiltration and conductivity adjusted below 5 mS to approximately 4 mS. The harvest was concentrated 6 to 9 fold and diafiltered against 20 mM Na-Phosphate, pH 7.5. The resulting volume was between 2.2 and 3.3 litres.

After the harvest was concentrated by ultrafiltration and dialysed, EPO was purified by the following sequence of chromatography steps.

Of course, also other EPO-producing clones and cell lines can be used, e. g. as disclosed in EP-A-0 148 605 and EP-A-0 205 564. The same applies to the culture conditions, also here several protocols and conditions are described in the patent literature as well as the scientific literature.

### Step a) Anion Exchange Chromatography as capture step

200 ml of Q Sepharose XL resin (Amersham Biosciences, Germany) were packed in a XK50 column (Amersham Biosciences, Germany). The capture was performed at 2-8°C.

The following buffers were used:
buffer A: 20 mM Na-Phosphate pH 7.5
buffer B: 300 mM NaCl, 20 mM Na-Phosphate pH 7.5

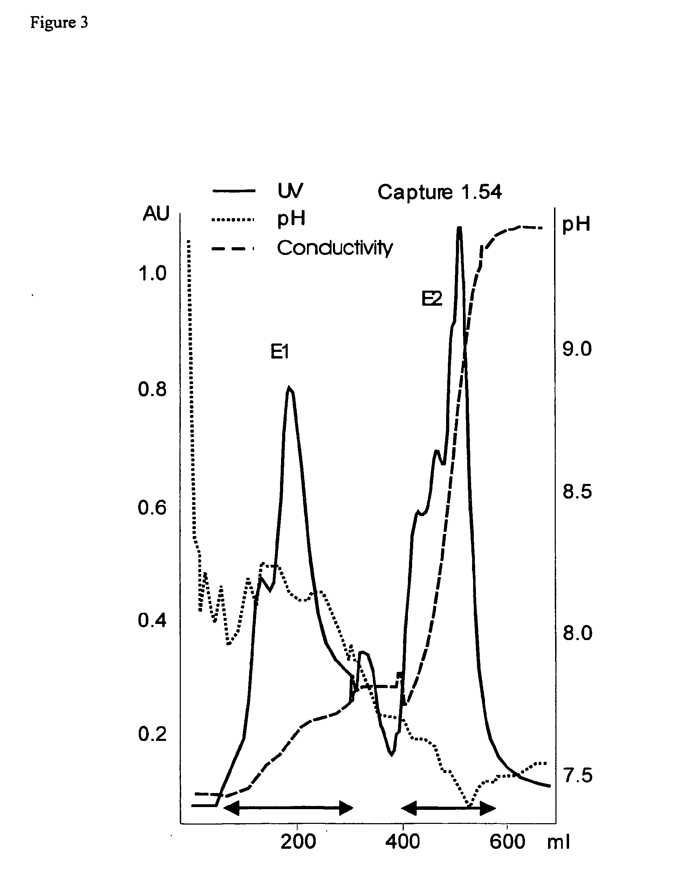

Processing of this chromatography step was carried out as follows:
Equilibration of the column with 10 CV buffer A at 70 ml/min (220 cm/h)
Loading of sample at 70 ml/min (220 cm/h)
Wash step with buffer A with 2 CV at 70 ml/min (220 cm/h)
Elution with step gradient: 100 % buffer B at 25 ml/min (80 cm/h) (Peak E1 of Figure 3)

The results are given in Table 1.

**Table 1 Result of capture Step. For elution profile see Figure 3**

| | Sample name | Volume [L] | EPO [mg/l] | EPO [mg] | Yield % |
|---|---|---|---|---|---|
| Step 1 (Start) | | | | | |
| Start material: | A | 3.14 | 110.48 | 346.9 | |
| | | | | | |

| Step 2 (Load) | | | | | |
|---|---|---|---|---|---|
| Flow through | D | 3.1 | 6.86 | 21.26 | 6 |
| Wash | W | 0.5 | 9.27 | 4.63 | 1.3 |
| | | | | | |

| Step 3 (Elution) | | | | | |
|---|---|---|---|---|---|
| Peak | E1 | 0.250 | 1097.1 | 274.2 | 79 |
| | E2 | 0.090 | 15.90 | 1.43 | 0.4 |
| | | | | | |

| Step 4 (Wash) | | | | | |
|---|---|---|---|---|---|
| Peak | E3 | 0.180 | 1.64 | 0.29 | 0.1 |
| | | | | | |
| Total eluted | | | | 301.81 | 87 |

### Step b) Hydrophobic Interaction Chromatography (HIC)

400 ml Butyl-Sepharose FF (Amersham Biosciences, Germany) were packed in a XK-50 column (Amersham Biosciences, Germany). Purification was performed at ambient temperature.

The following buffers were used:
buffer A: 4 M NaCl, 20 mM Na-Phosphate pH 7.5
buffer B: 5 mM CaCl₂, 20 mM Tris HCl pH 6.9
buffer C: 2 M NaCl, 10% Isopropanol, 20 mM Na-Phosphate pH 7.5
buffer D: 0,75 M NaCl, 5 mM CaCl₂, 20 mM Tris HCl pH 6.9
buffer E: 0,75 M NaCl, 5 mM CaCl₂, 50% Isopropanol, 20 mM Tris HCl pH 6.9

Processing of this chromatography step was carried out as follows:

Capture eluate from step a) was diluted with 85% (v/v) of buffer A. 10 % Isopropanol (v/v) were added in order to establish buffer conditions comparable to buffer C. The conductivity was between 95 and 105 mS/cm.

| | |
|---|---|
| Equilibration: | 10 CV buffer C at 25 ml/min (80 cm/h) |
| Loading: | at 25 ml/min (80 cm/h). |
| Wash: | 3 CV of buffer C at 25 ml/min |
| Elution (step gradient): | 3 CV of 38% buffer E and 62% buffer D at 25 ml/min (80 cm/h) |

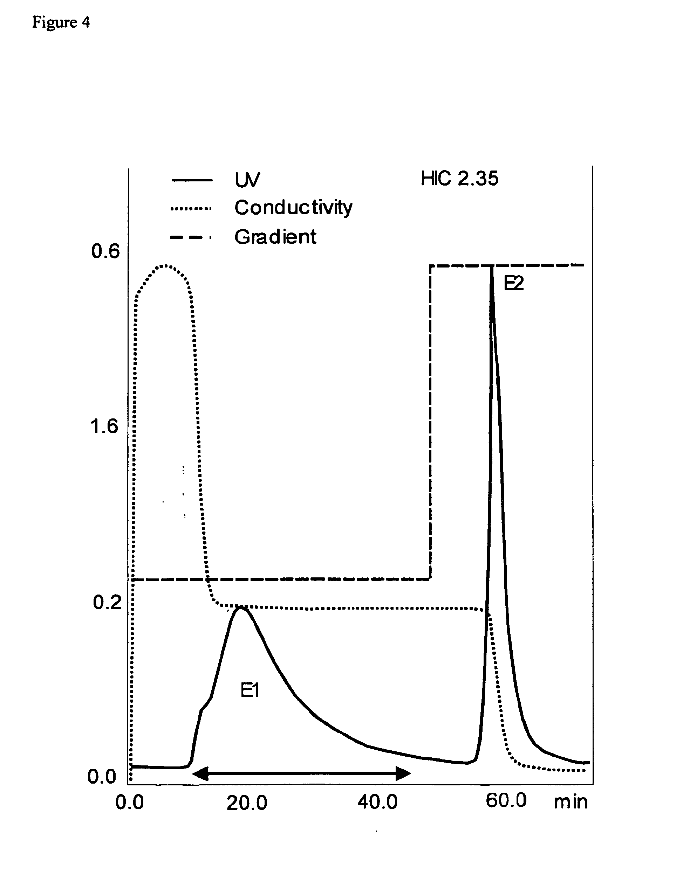

Peak E1 (see Figure 4) was collected in 2.5 CV (1000 ml) ice cold buffer B and was processed immediately on the affinity hydroxyapatite column.

The results of this step are given in Table 2.

**Table 2 Results of the HIC step. For elution profile see Figure 4**

| | Sample name | Volume [L] | EPO [mg/l] | EPO [mg] | Yield % |
|---|---|---|---|---|---|
| Step 1 (Start) | | | | | |
| Start material: | A | 0.509 | 453.1 | 230.6 | |
| | | | | | |

| Step 2 (Load) | | | | | |
|---|---|---|---|---|---|
| Flow through | D | 0.510 | 2.49 | 1.26 | |
| Wash | W | 2.000 | 0.58 | 1.16 | |
| | | | | | |

| Step 3 (Elution) | | | | | |
|---|---|---|---|---|---|
| Peak | E1 | 1.875 | 111.5 | 209.06 | 90 |
| | | | | | |

| Step 4 (Wash) | | | | | |
|---|---|---|---|---|---|
| Peak | E2 | 0.250 | 195.8 | 48.95 | 21 |
| | | | | | |
| Total eluted | | | | 260.43 | 111 |

### Step c): Affinity Chromatography with HAP

135 ml of Macro-Prep (Ceramic), Typ I, 40 µm (BioRad, Germany) were packed in a XK 26/40 (Amersham Biosciences, Germany). The process was performed at ambient temperature.

The following buffers were used:
buffer A: 0.25 M NaCl, 5 mM CaCl₂, 9% Isopropanol, 20 mM Tris pH 6.9
buffer B: 20 mM Tris HCl pH 6.9
buffer D: 100 mM K₂HPO₄, 10 mM Tris, pH 6.9

Processing of this chromatography step was carried out as follows:

| | |
|---|---|
| Equilibration: | 11.5 CV buffer A at 10 ml/min (113 cm/h) |
| Loading: | at 7 ml/min (80 cm/h) |
| Wash step 1: | 5.75 CV buffer A at 7 ml/min (80 cm/h) |
| Wash step 2: | 1.15 CV buffer B at 7 ml/min (80 cm/h) |
| Elution: | buffer B, D: gradient from 0% to 40% buffer D in 4.6 CV at 7 ml/min. |

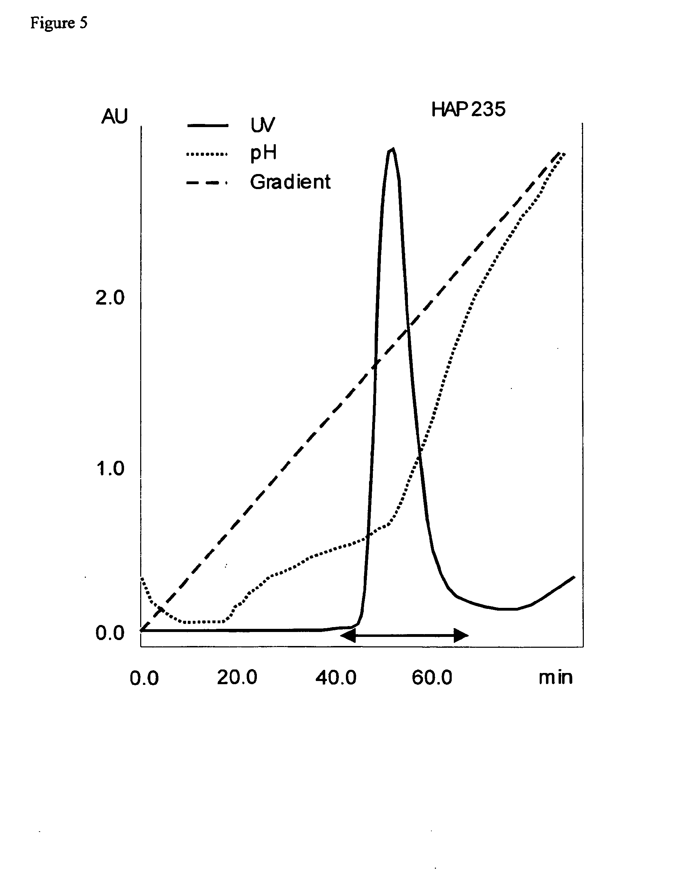

Results: Erythropoietin elutes in one peak (Figure 5), which was processed further on a second anion exchange column.

The results of this step are given in Table 3:

**Table 3 Results of the HAP step. For elution profile see Figure 5**

| | Sample name | Volume [L] | EPO [mg/l] | EPO [mg] | Yield % |
|---|---|---|---|---|---|
| Step 1 (Start) | | | | | |
| Start material: | A | 1.79 | 111.5 | 199.6 | |
| | | | | | |

| Step 2 (Load/Wash) | | | | | |
|---|---|---|---|---|---|
| Flow through | D | 1.80 | 0.85 | 1.53 | 1 |
| | W1 | 0.75 | 0.91 | 0.68 | 0.5 |
| | W2 | 0.15 | 0.51 | 0.08 | 0.05 |
| | | | | | |

| Step 3 (Elution) | | | | | |
|---|---|---|---|---|---|
| Peak | E1 | 0.200 | 745.9 | 149.2 | 75 |
| | | | | | |
| Total: | | | | 151 | 77 |

### Step d) Anion exchange Chromatography

This step was performed with a prepacked Resource Q 1ml (1ml Source 15Q, Amersham Biosciences, Germany). The column run was performed at ambient temperature. Alternatively, an ion exchange membrane was used (Sartobind Membraneadsorber (MA) Q100 (Sartorius, Germany).

The following buffers were used:
buffer A: 10 mM Na-Phosphate pH 7.4
buffer B: 1M NaCl, 20 mM Na-Phosphate pH 7.4
buffer C: 20 mM Na-Acetate pH 4.0

Processing of this chromatography step was carried out as follows:

| | |
|---|---|
| Equilibration: | 10 CV buffer A at 1 ml/min |
| Loading: | at 1 ml/min (186 cm/h) |
| Wash: | 10 CV buffer A at 1 ml/min |
| Acidic wash: | 2 CV buffer C at 1 ml/min |
| Wash: | 10 CV buffer A at 1 ml/min |
| Step-elution: | 1 CV 50% buffer B at 1 ml/min |

The results are given in Table 4:

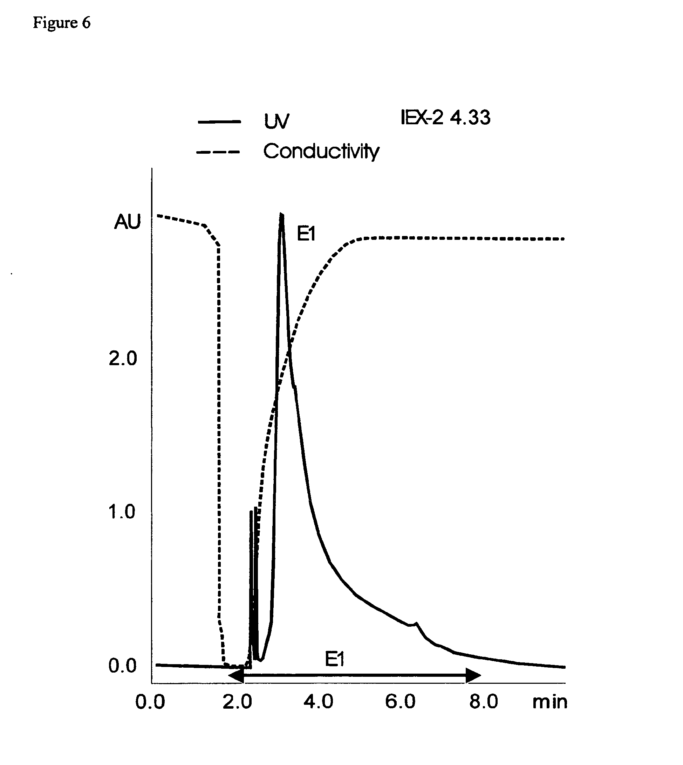

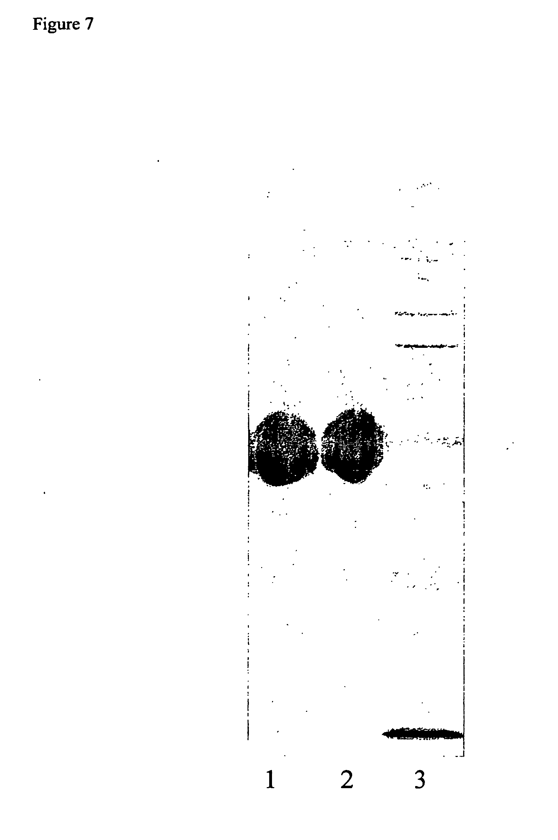

**Table 4 Results of the second anion exchange step. For elution profile see Figure 6**

| | Sample name | Volume [L] | EPO [mg/l] | EPO [mg] | Yield % |
|---|---|---|---|---|---|
| Step 1 (Start) | | | | | |
| Start material: | Pool1 | 0.0067 | 854.07 | 5.72 | |
| | | | | | |

| Step 2 (Load) | | | | | |
|---|---|---|---|---|---|
| Flow through | D | 0.007 | 0.48 | 0.003 | 0 |
| | | | | | |

| Step 3 (acidic wash) | | | | | |
|---|---|---|---|---|---|
| Peak | AW | 0.0035 | 191.24 | 0.67 | 11 |
| | | | | | |

| Step 4 (Elution) | | | | | |
|---|---|---|---|---|---|
| Peak | E | 0.0055 | 670.6 | 3.68 | 64 |
| | | | | | |
| Total eluted: | | | | 4.35 | 74 |

Peak E1 of Figure 6 was then analysed by SDS-PAGE-Gelelectrophoresis and subsequent Coomassie staining. As can be see from Figure 6, erythropoietin was eluted in a single step at high purity, comparable to that of the Ph. Eur. BRP standard.

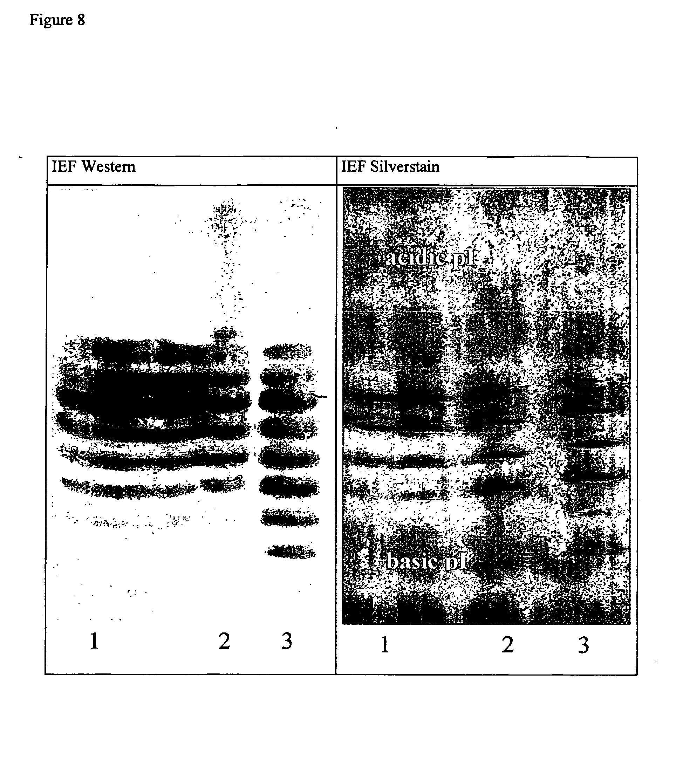

Subsequently Peak E1 of Figure 6 was analysed with respect to its isoform content by IEF (Isoelectric focusing gel electrophoresis was carried out on a preacst gel (Amersham Biosciences) using the Phast system (Amersham Biosciences) and applying a pH gradient of pH 3.5 to 6.5) (Figure 8). EPO isoforms were visualised either by Western blotting or Silver staining. The acidic wash led to a reduction of basic pI isoforms, which yielded an isoform profile, closely resembling that of the Ph. Eur. BRP standard (Figure 8).

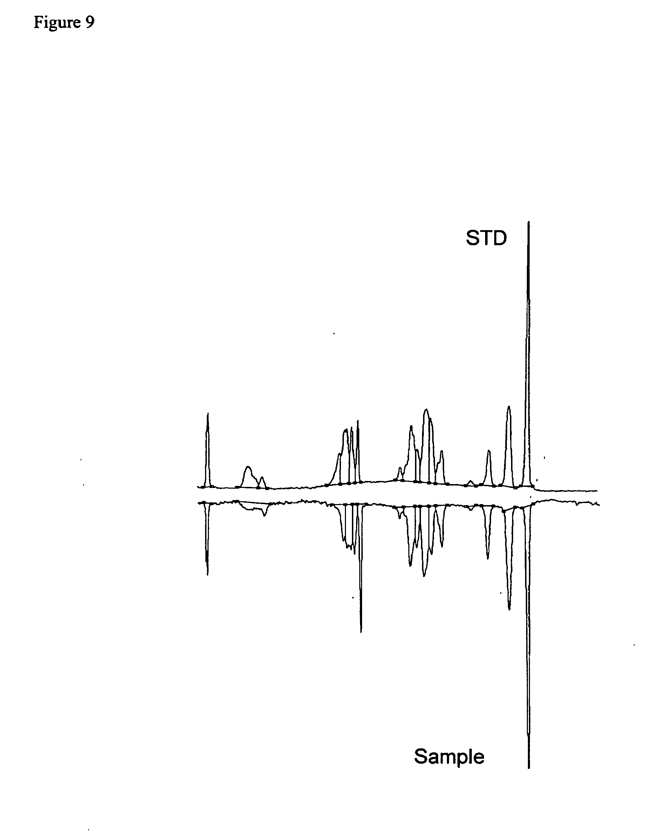

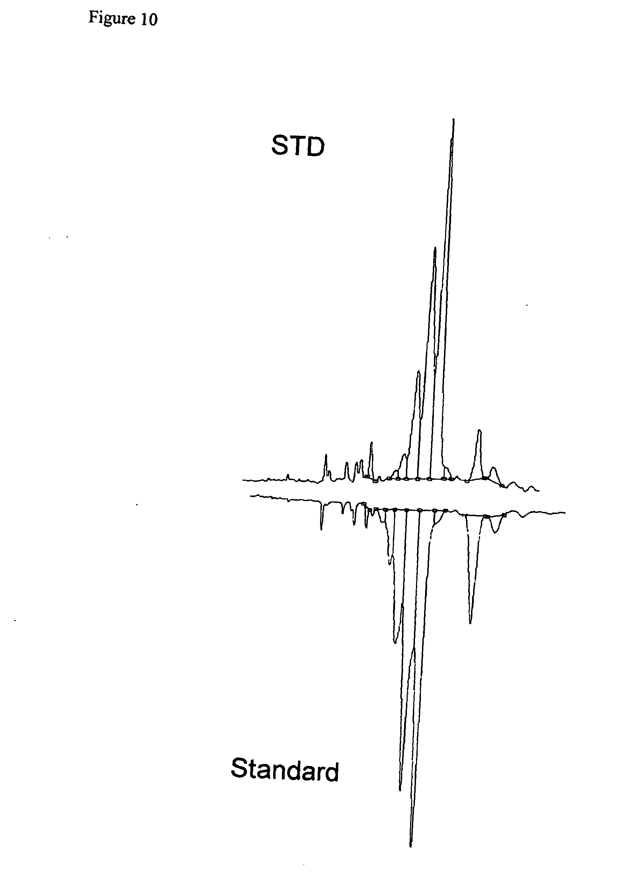

This was further confirmed by DEAE HPLC analysis of AB (2-aminobenzamide) labelled native (Figure 9) and neutral (Figure 10) sugar oligos, which were released from purified erythropoietin (Kanazawa (1999) Biol. Pharm. Bull 22 (4) 339-346).

In another embodiment of the invention, the DSP process, as exemplified for erythropoietein purification can comprise an acidic wash step at the first anion exchange chromatography step. This is exemplified as follows.

### Step a) anion exchange chromatography as capture step with an acidic wash step

Culture extracts were prepared as outlined above. The same volume was applied to the anion exchange column.

170 ml of Q Sepharose XL resin (Amersham Biosciences, Germany) were packed in a XK50 column (Amersham Biosciences, Germany). The capture was performed at ambient temperature.

The following buffers were used:
buffer A1: 20 mM Na-Phosphate pH 7.5
Buffer A2: 33 mM Na-Acetate pH 4.0
Buffer B: 300 mM NaCl, 20 mM Na-Phosphate pH 7.5

Processing of this chromatography step was carried out as follows:
Equilibration of the column with 5 CV buffer A1 at 25 ml/min (80 cm/h).
Loading of sample at 25 ml/min (80 cm/h).
Wash step with buffer A1 with 2 CV at 25 ml/min (80 cm/h)
Wash step with buffer A2 with 3 CV at 25 ml/min (80 cm/h)
Elution with step gradient with buffer B at 25 ml/min (80 cm/h)

Step elution resulted in 2 peaks (E2 and E3). The results are given in Table 5.

**Table 5 Data from the capture step with an acidic wash step**

| Run 1.21 | Volume [ml] | Erythropoietin conc. [mg/l] | Erythropoietin total [mg] | Yield % |
|---|---|---|---|---|
| Load | 760 | 81.8 | 62.2 | 100 |
| Flow through | 800 | 1 | 0.8 | 1.3 |
| Acidic Wash: Peak E1 | 100 | 55.6 | 5.6 | 9 |
| Elution peak E2 | 59 | 648.9 | 38.3 | 61.6 |
| Elution peak E3 | 35 | 40.5 | 1.4 | 2.2 |
| Total eluted | | | | 74.1 |

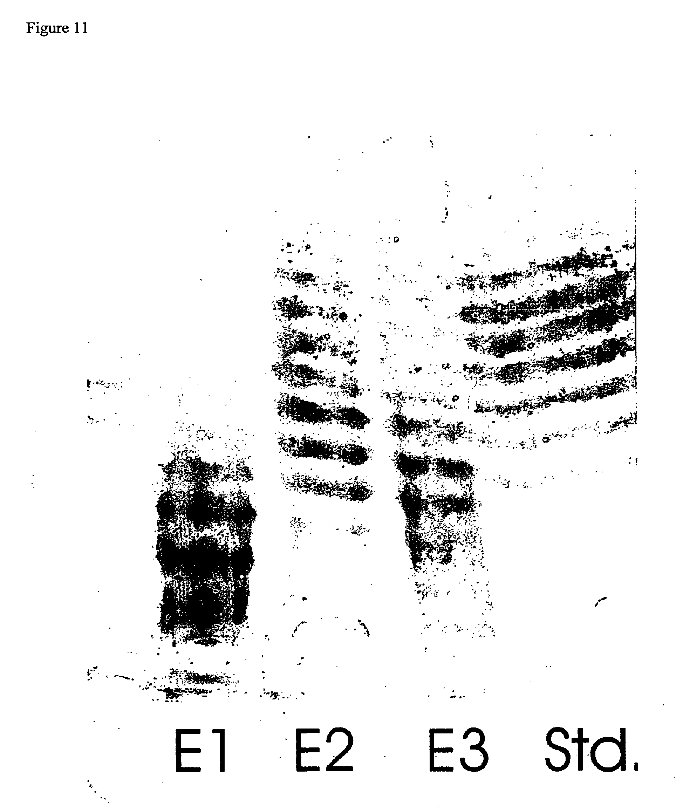

As can be seen from Figure 11, the acidic wash step with 33 mM Na-acetate pH 4.0 leads to the loss of some material (E1). It allows, however, for discriminating erythropoietin isoforms (E2 and E3), which resemble that of the Ph. Eur. BRP standard (Std), and the more basic erythropoietin isoforms (E1). This yields an adjustment of the erythropoietin isoform profile to that of the BRP standard already at the capture step.

It was also tested which salt buffer conditions are optimal if an acidic wash step is carried out at the second anion exchange chromatography step.

### Optimisation of the acidic wash step at the second anion exchange step

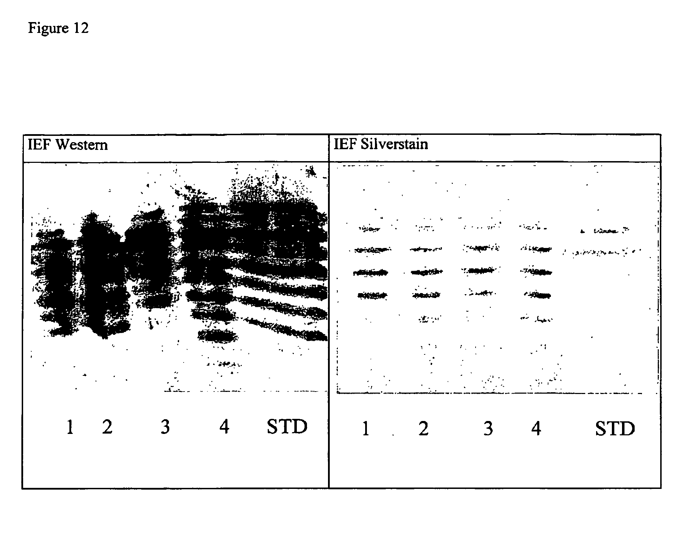

To optimise the buffer conditions of the acidic wash step at the second anion exchange chromatography column, buffers were prepared containing 5, 10 or 20 mM Na-Acetate pH 4.0. The purification step was carried out as described above for step d). Afterwards eluates were analysed with respect to their erythropoietein isoform content by IEF (Figure 12).

Concentrations ranging of 5 mM, 10 mM and 20 mM Na-Acetate pH 4.0 yielded increasing material in the acidic wash (1%, 6.3% and 10%) and a shift towards a more acidic isoform profile of the eluted erythropoietin. As shown in Figure 12, at a 20 mM Na-Acetate pH 4.0 wash, the erythropoietein isoform profile closely resembles that of the Ph. Eur. BRP standard. Therefore this concentration is preferred for purification of highly sialylated forms of EPO.

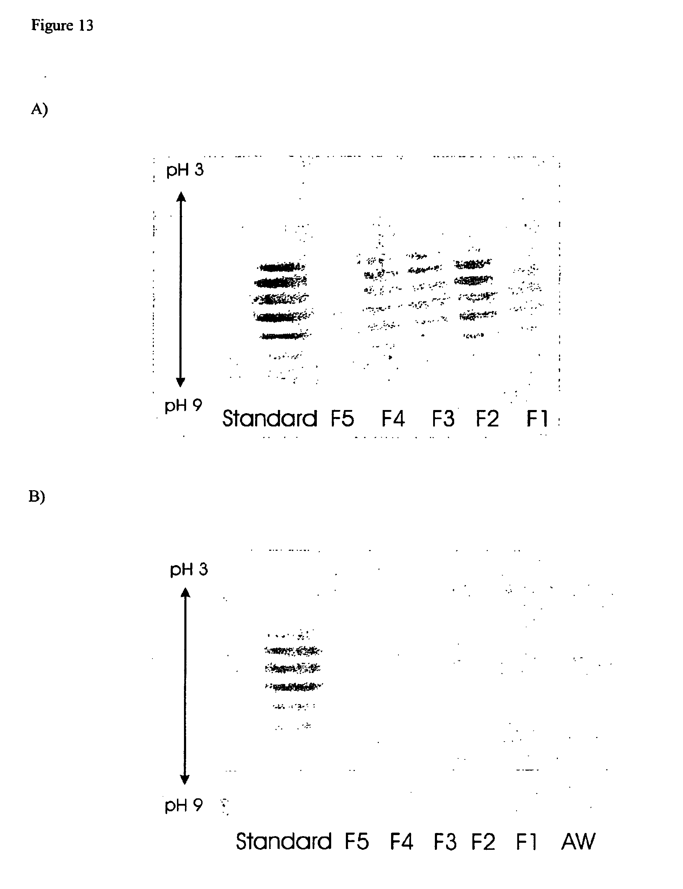

Erythropoietin, which was exposed to an acidic wash step at the capture, was processed through the 4-step purification protocol with or without a second acidic wash step at the second anion exchange chromatography step. Isoforms isolated by the second anion exchange chromatography step were analysed by IEF followed by Western Blotting (see Figure 13). Purification sequence was otherwise identical to the procedure as described above.

Figure 13 shows that isoforms of erythropoietin purified without an additional acidic wash step at the second anion exchange step contain most of the isoform bands as the Ph. Eur. BRP erythropoietin standard (Figure 13A). It could further be demonstrated that a second acidic wash step leads to a selective enrichment of more acidic isoforms, represented by the 3 - 4 most acidic isoforms (Figure 13B). The second acidic wash removes most of the remaining basic erythropoietin isoforms.

Thus, a 2-step acidic wash procedure has been shown to be another means by which a strong relative enrichment of acidic isoforms can be achieved.

Thus, the present invention allows for purification of a glycoprotein with a specific isoform composition, rich in acidic pI isoforms. This is achieved by combining favourable cell culture conditions and new purification techniques. Based on a cell clone, which was selected for the production of highly sialylated product, i) culture conditions for the production of the highly sialylated isoforms were identified to be optimal in the range of pH 7.2 to pH 7.5 and ii) at least one acidic wash step at one of anion exchange steps during purification was found to provide measures for further selective enrichment of the more acidic isoforms.

### Figure Legends

### Figure 1:

Isoform distribution of the Ph. Eur. BRP standard and of erythropoietin, which was produced by CHO cultures at pH 6.9, pH 7.2 and pH 7.5. Erythropoietin was purified according to a 2-step purification protocol (capture with Q-Sepharose XL and salt step elution followed by a HIC step with butyl-sepharose FF as set out in the examples) and analysed by capillary electrophoresis (CE). The lower peak numbers present the isoforms of basic, the higher peak numbers the isoforms of acidic pI. High culture pH yields an increased degree of acidic erythropoietin isoforms. The peak numbers correspond to the Ph. Eur. annotation.

### Figure 2:

CHO cultures, which produce erythropoietin, were grown at pH 6.9, pH 7.2 and pH 7.5. Cells were growing best at lower pH (pH 6.9) and had the best productivity at higher pH (pH 7.5). The glycoform-profile was better at higher pH (pH 7.2 and pH 7.5, see Figure 1). The culture-conditions of pH 7.2 provide for good cell-growth, high product sialylation and good final product yield.

### Figure 3:

Elution profile of step a) of the purification sequence. Peak E1 was collected, peak E2 was recorded during cleaning.

### Figure 4:

Elution profile of step b) of the purification sequence. Peak E1 was collected, peak E2 was recorded during cleaning.

### Figure 5:

Elution profile of step c) of the purification sequence. The elution peak was collected as indicated by the arrow.

### Figure 6:

Elution profile of step d) of the purification procedure. Peak E1 was collected as indicated by the arrow.

### Figure 7:

Coomassie Blue stained SDS-PAGE-Gel analysis shows comparable purity between the erythropoietin after step d) (lane 1) and the Ph. Eur. BRP standard (lane 2). Lane 3 is a marker.

### Figure 8:

Isoelectric focussing of Ph. Eur. BRP standard (lane 1), eluted purified erythropoietin from step d) (lane 2) and load (lane 3) of this chromatography step. Detection was achieved by Western Blotting (left panel) and Silver staining (right panel). The more basic pI isoforms in the load (lane 3) were significantly removed by the acidic wash. This yields a product (lane 2) with an isoform profile closely comparing to that of the Ph. Eur. BRP standard (lane 1).

### Figure 9:

Mirror image comparison of the native erythropoietin sugar oligos released from the purified erythropoieting after step d) and the one released form the Ph. Eur. BRP standard. The oligos were AB-labelled and separated by HPLC on a DEAE column. One acidic wash at the second anion-exchange chromatography step yielded an oligosaccharide distribution of the sample, which compares to that of the Ph. Eur. BRP standard (STD).

### Figure 10:

Mirror image comparison of the neutral erythropoietin sugar oligos released from the purified erythropoietin after step d) and the ones released form the Ph. Eur. BRP standard. The acidic wash in the second capture yields a distribution profile of neutral oligosaccharides, which has high similarity to that of the Ph. Eur. BRP standard (STD).

### Figure 11:

Erythropoietin isoform composition in the acidic wash fraction (E1) and the elution peaks (E2 and E3) at the capture step was analysed by IEF. Protein isoforms were visualised by Western Blotting. The acidic wash with 33 mM Na acetate, pH 4.0 allows to discriminate erytropoietin isoforms, which resemble that of the Ph. Eur. BRP standard (Std.) and the more basic isoforms. This yields an adjustment of the erythropoietin isoform profile to that of the BRP standard already at the capture step.

### Figure 12:

Analysis of the load of the second anion-exchange column (lane 4) and of the elutions after acidic washes with 20 mM (lane 3), 5 mM (lane 2) and 10 mM (lane 1) Na-Acetate pH 4.0 with respect to the isoform distribution by IEF. Protein isoforms were visualised by Western Blotting (left panel) or Silver staining (right panel). After an acidic wash at the second anion exchange step, eluates contained significantly less isoforms of basic pI compared to the load (lane 4). The buffer with 20 mM Na-Acetate pH 4.0 yielded the strongest reduction. The isoform profile is comparable to that of the BRP standard (STD).

### Figure 13:

Analysis of the erythropoietin isoforms in the eluate of the second anion exchange step as analyed by IEF. Proteins were visualised by Western blotting. A) Purification without an acidic wash step after the second ion exchange column. B) Purification with an additional acidic wash step after the second ion exchange column. The eluate was collected in 5 fractions (F1 to F5). An acidic wash had been performed in A) and B) at the first anion exchange chromatography capture step.

The product without a second acidic wash (A) contains most of the isoforms of the Ph. Eur. Standard (Standard). Product with a second acidic wash (B) is selectively enriched in the more acidic pI isoforms, represented by the 3-4 most acidic isoforms.

## Claims

1. A method of purifying acidic erythropietin isoforms from a complex protein mixture, **characterised in that**
the method comprises two anion exchange chromatography steps, which are separated by chromatography steps based on a principle other than anion exchange chromatography, and at least one acidic wash step at one of the anion exchange steps.

2. The method according to claim 1, **characterised in that**
the method comprises the following steps:
a) a first anion exchange chromatography step as a capture step and an optional acidic wash step,
b) hydrophobic interaction chromatography,
c) affinity chromatography,
d) a second anion exchange chromatography step and an acidic wash step.

3. The method according to claim 2, **characterised in that**
elution in substeps a) to d) is carried out by step or gradient elution.

4. The method according to claim 2 or 3, **characterised in that**
substeps a) and d) are performed with anion exchange resins or membranes that contain Diethylaminoethyl-groups (DEAE), quaternary aminoethyl-groups (QAE), quaternary ammonium-groups (Q), Dimethylaminoethyl-groups (DMAE) and/or Trimethylaminoethyl-groups (TMAE) as functional groups.

5. The method according to claim 4, **characterised in that**
substep a) is performed with commercially available Q-Sepharose XL resin.

6. The method according to claim 4, **characterised in that**
substep d) is performed with commercially available Resource Q resin

7. The method according to claim 2 or 3, **characterised in that**
substep b) is performed with resins that contain Butyl-, Phenyl-, Propyl- and/or Octyl-groups as functional groups.

8. The method according to claim 7, **characterised in that**
substep b) is performed with commercially available Butyl-Sepharose FF resin.

9. The method according to claim 2 or 3, **characterised in that**
substep c) is performed with affinity resins such as hydroxyapatite, Ni-NTA, Talon, dye chromatography resins such as Dye- or Blue-Sepharose, antibody-affinity resins, lectine affinity resins and/or peptide-ligand-affinity resins.

10. The method according to claim 9, **characterised in that**
substep c) is performed with commercially available hydroxyapatite Macro-Prep Typ I resin.

11. The method according to any of claims 1 to 10, **characterised in that**
the buffer for the acidic wash step has a pH between 2.0 and 5.0, preferably between 2.5 and 4.5 and more preferably between 3.0 and 4.0.

12. The method according to any of claims 1 to 11, **characterised in that**
the buffer for the acidic wash step contains Na-Actetate, Na-Citrate, HEPES, PIPES, MES and/or MOPS as buffering agents.

13. The method according to any of claims 1 to 12, **characterised in that**
the buffer for the acidic wash step in the capture step contains between 5 and 50 mM Na-Actetate with a pH between 3.0 and 4.0, preferably between 10 and 40 mM Na-Actetate with a pH between 3.0 and 4.0, even more preferably between 15 and 35 mM Nac-Acetate with a pH between 3.0 and 4.0 and most preferably 33 mM Na-Actetate pH 4.0.

14. The method according to any of claims 1 to 12, **characterised in that**
the buffer for the acidic wash step in the second anion exchange chromatography step contains between 5 and 50 mM Na-Actetate with a pH between 3.0 and 4.0, preferably between 10 and 40 mM Na-Actetate with a pH between 3.0 and 4.0, even more preferably between 15 and 30 mM Nac-Acetate with a pH between 3.0 and 4.0 and most preferably 20 mM Na-Actetate pH 4.0.

15. The method according to any of claims 1 to 14, **characterised in that** the erythropoietin is human recombinant erythropoietin.

16. Acidic erythropoietin isoforms purified by a method according to any of claims 1 to 14.

17. The acidic erythropoietin isoforms according to claim 16, wherein the erythropoietin is human recombinant erythropoietin.

## Patentansprüche

1. Verfahren zur Reinigung von sauren Erythropoietin-Isoformen aus einem komplexen Proteingemisch, **dadurch gekennzeichnet, dass**
das Verfahren zwei Anionenaustausch-Chromatographieschritte, die durch Chromatographieschritte basierend auf einem anderem Prinzip als Anionenaustausch-Chromatographie getrennt sind, und mindestens einen sauren Waschschritt in einem der Anionenaustauschschritte umfasst.

2. Verfahren nach Anspruch 1, **dadurch gekennzeichnet, dass**
das Verfahren folgende Schritte umfasst:
a) einen ersten Anionenaustausch-Chromatographieschritt als Capture-Schritt und einen optionalen sauren Waschschritt,
b) hydrophobe Interaktionschromatographie,
c) Affinitätschromatographie,
d) einen zweiten Anionenaustausch-Chromatographieschritt und einen sauren Waschschritt.

3. Verfahren nach Anspruch 2, **dadurch gekennzeichnet, dass**
die Elution in den Unterschritten a) bis d) als Stufen- oder Gradienten-Elution durchgeführt wird.

4. Verfahren nach Anspruch 2 oder 3, **dadurch gekennzeichnet, dass**
die Unterschritte a) und d) mit Anionenaustausch-Harzen oder -Membranen enthaltend Diethylaminoethyl-Gruppen (DEAE), quaternäre Aminoethyl-Gruppen (QAE), quaternäre Ammonium-Gruppen (Q), Dimethylaminoethyl-Gruppen (DMAE) und/oder Trimethylaminoethyl-Gruppen (TMAE) als funktionelle Gruppen durchgeführt werden.

5. Verfahren gemäß Anspruch 4, **dadurch gekennzeichnet, dass**
Unterschritt a) mit kommerziell erhältlichem Q-Sepharose XL-Harz durchgeführt wird.

6. Verfahren gemäß Anspruch 4, **dadurch gekennzeichnet, dass**
Unterschritt d) mit kommerziell erhältlichem Resource Q-Harz durchgeführt wird.

7. Verfahren gemäß Anspruch 2 oder 3, **dadurch gekennzeichnet, dass**
Unterschritt b) mit einem Harz enthaltend Butyl-, Phenyl-, Propyl- und/oder Octyl-Gruppen als funktionelle Gruppen durchgeführt wird.

8. Verfahren gemäß Anspruch 7, **dadurch gekennzeichnet, dass**
Unterschritt b) mit kommerziell erhältlichem Butyl-Sepharose FF-Harz durchgeführt wird.

9. Verfahren gemäß Anspruch 2 oder 3, **dadurch gekennzeichnet, dass**
Unterschritt c) mit Affinitäts-Harzen wie Hydroxyapatit-, Ni-NTA-, Talon-, Farbstoff-Chromatographieharzen wie Dye- oder Blue-Sepharose, Antikörper-Affinitätsharzen, Lectin-Affinitätsharzen und/oder Peptid-Ligand-Affinitätsharzen durchgeführt wird.

10. Verfahren gemäß Anspruch 9, **dadurch gekennzeichnet, dass**
Unterschritt c) mit kommerziell erhältlichem Hydroxyapatit Macro-Prep Typ 1-Harz durchgeführt wird.

11. Verfahren gemäß einem der Ansprüche 1 bis 10, **dadurch gekennzeichnet, dass**
der Puffer für den sauren Waschschritt einen pH zwischen 2,0 und 5,0, bevorzugt zwischen 2,5 und 4,5 und besonders bevorzugt zwischen 3,0 und 4,0 besitzt.

12. Verfahren gemäß einem der Ansprüche 1 bis 11, **dadurch gekennzeichnet, dass**
der Puffer für den sauren Waschschritt Na-Acetat, Na-Citrat, HEPES, PIPES, MES und/oder MOPS als Pufferagenzien enthält.

13. Verfahren gemäß einem der Ansprüche 1 bis 12, **dadurch gekennzeichnet, dass**
der Puffer für den sauren Waschschritt im Capture-Schritt zwischen 5 und 50 mM Na-Acetat mit einem pH zwischen 3,0 und 4,0, bevorzugt zwischen 10 und 40 mM Na-Acetat mit einem pH zwischen 3,0 und 4,0, besonders bevorzugt zwischen 15 und 35 mM Na-Acetat mit einem pH zwischen 3,0 und 4,0 und am meisten bevorzugt 33 mM Na-Acetat pH 4,0 enthält.

14. Verfahren gemäß einem der Ansprüche 1 bis 12, **dadurch gekennzeichnet, dass**
der Puffer für den sauren Waschschritt im zweiten Anionenaustausch-Chromatographieschritt zwischen 5 und 50 mM Na-Acetat mit einem pH zwischen 3,0 und 4,0, bevorzugt zwischen 10 und 40 mM Na-Acetat mit einem pH zwischen 3,0 und 4,0, besonders bevorzugt zwischen 15 und 30 mM Na-Acetat mit einem pH zwischen 3,0 und 4,0 und am meisten bevorzugt 20 mM Na-Acetat pH 4,0 enthält.

15. Verfahren gemäß einem der Ansprüche 1 bis 14, **dadurch gekennzeichnet, dass** das Erythropoietin humanes rekombinantes Erythropoietin ist.

16. Saure Erythropoietin-Isoformen gereinigt durch ein Verfahren gemäß einem der Ansprüche 1 bis 14.

17. Saure Erythropoietin-Isoformen gemäß Anspruch 16, wobei das Erythropoietin humanes rekombinantes Erythropoietin ist.

## Revendications

1. Une méthode de purification des isoformes acides de l'érythropoïétine à partir d'un mélange complexe de protéines **caractérisée en ce que**
la méthode comprend deux étapes de chromatographie d'échange d'anions, qui sont séparées par des étapes de chromatographie basées sur une autre principe que la chromatographie d'échange d'anions, et au moins une étape de lavage acide lors d'une des étapes d'échange d'anions.

2. La méthode selon la revendication 1, **caractérisée en ce que**
la méthode comprend les étapes suivantes :
a) une première étape de chromatographie d'échange d'anions comme étape de capture et une étape de lavage acide optionnelle,
b) une chromatographie d'interaction hydrophobe,
c) une chromatographie d'affinité,
d) une seconde une étape de chromatographie d'échange d'anions et une étape de lavage acide.

3. La méthode selon la revendication 2, **caractérisée en ce que**
l'élution lors des sous-étapes a) à d) est effectuée par élution à étapes ou gradient d'élution.

4. La méthode selon la revendication 2 ou 3, **caractérisée en ce que**
les sous-étapes a) et d) sont effectuées en utilisant des résines ou des membranes d'échange d'anions qui contiennent des groupes diéthylaminoéthyle (DEAE), des groupes aminoéthyle quaternaires (QAE), des groupes ammonium quaternaires (Q), des groupes diméthylaminoéthyle (DMAE) et/ou des groupes triméthylaminoéthyl (TMAE) comme groupes fonctionnels.

5. La méthode selon la revendication 4, **caractérisée en ce que**
la sous-étape a) est réalisée en utilisant une résine Q-Sepharose XL disponible dans le commerce.

6. La méthode selon la revendication 4, **caractérisée en ce que**
la sous-étape d) est réalisée en utilisant une résine Resource Q disponible dans le commerce.

7. La méthode selon la revendication 2 ou 3, **caractérisée en ce que**
la sous-étape b) est réalisée en utilisant des résines contenant des groupes butyle, phényle, propyle et/ou octyle comme groupes fonctionnels.

8. La méthode selon la revendication 7, **caractérisée en ce que**
la sous-étape b) est réalisée en utilisant une résine Butyl-Sepharose FF disponible dans le commerce.

9. La méthode selon la revendication 2 ou 3, **caractérisée en ce que**
la sous-étape c) est réalisée en utilisant des résines d'affinité telles que l'hydroxyapatite, la Ni-NTA, la Talon, des résines de chromatographie sur colorant telles que la Dye- ou la Blue-Sepharose, des résines d'affinité d' anticorps, des résines d'affinité de lectine et /ou des résines d'affinité de peptide-ligand.

10. La méthode selon la revendication 9, **caractérisée en ce que**
la sous-étape c) est réalisée en utilisant une résine hydroxyapatite Macro-Prep Type I disponible dans le commerce.

11. La méthode selon n'importe laquelle des revendications 1 à 10, **caractérisée en ce que** le tampon pour l'étape de lavage acide a un pH entre 2.0 et 5.0, de préférence entre 2.5 et 4.5 et plus préférentiellement entre 3.0 et 4.0.

12. La méthode selon n'importe laquelle des revendications 1 à 11, **caractérisée en ce que** le tampon pour l'étape de lavage acide contient de l'acétate de Na, du citrate de Na, de l'HEPES, du PIPES, du MES et/ou du MOPS comme agents tampons.

13. La méthode selon n'importe laquelle des revendications 1 à 12, **caractérisée en ce que** le tampon pour l'étape de lavage acide lors de l'étape de capture contient entre 5 et 50 mM d'acétate de Na ayant un pH compris entre 3.0 et 4.0, de préférence entre 10 et 40 mM d'acétate de Na ayant un pH compris entre 3.0 et 4.0, plus préférentiellement entre 15 et 35 mM d'acétate de Na ayant un pH compris entre 3.0 et 4.0 et au mieux 33 mM d'acétate de Na à pH 4.0.

14. La méthode selon n'importe laquelle des revendications 1 à 12, **caractérisée en ce que** le tampon pour l'étape de lavage acide lors de la seconde étape de chromatographie d'échange d'anions contient entre 5 et 50 mM d'acétate de Na ayant un pH compris entre 3.0 et 4.0, de préférence entre 10 et 40 mM d'acétate de Na ayant un pH compris entre 3.0 et 4.0, plus préférentiellement entre 15 et 30 mM d'acétate de Na ayant un pH compris entre 3.0 et 4.0 et au mieux 20 mM d'acétate de Na à pH 4.0.

15. La méthode selon n'importe laquelle des revendications 1 à 14, **caractérisée en ce que** l'érythropoïétine est l'érythropoïétine recombinante humaine.

16. Les isoformes acides de l'érythropoïétine purifiées par une méthode selon n'importe laquelle des revendications 1 à 14.

17. Les isoformes acides de l'érythropoïétine selon la revendication 16, dans lesquelles l'érythropoïétine est l'érythropoïétine recombinante humaine.
